# EUROPEAN PATENT SPECIFICATION

(11) **EP 1 960 012 B1**
(45) Date of publication and mention of the grant of the patent: **25.08.2010**
(21) Application number: 06845320.8
(22) Date of filing: 13.12.2006
(51) Int. Cl.: A61L 31/02, A61L 31/18, A61M 25/01, A61M 25/09

(54) **IMPLANTABLE MEDICAL DEVICE USING PALLADIUM**
IMPLANTIERBARE MEDIZINISCHE VORRICHTUNG MIT PALLADIUM
DISPOSITIF MÉDICAL IMPLANTABLE UTILISANT DU PALLADIUM

(30) Priority: 13.12.2005 US 749927 P
(43) Date of publication of application: 27.08.2008
(73) Proprietor: Cook Incorporated, Bloomington, Indiana 47402-0489 (US)
(72) Inventor: CARLSON, James, M., Bloomington, IN 47404 (US); PATTERSON, Donald, F., Bloomington, IN 47403 (US)
(74) Representative: Garratt, Peter Douglas
(86) International application number: PCT/US2006/047465
(87) International publication number: WO 2007/070544

(56) References cited:
- EP-A- 0 744 186
- WO-A-00/61203
- WO-A-2006/036786
- US-A- 5 019 337
- US-A- 6 117 157
- US-A1- 2003 044 307

## Description

### Technical Field

The present invention generally provides an implantable medical device for positioning in a body passage or lumen that is made at least in part of palladium for its tensile strength, radiopacity and MRI compatibility.

### Background of the Invention

It has become common to treat a variety of medical conditions by temporarily or permanently introducing an implantable medical device partly or completely into the esophagus, trachea, colon, biliary tract, urinary tract, vascular system or other location within a human or veterinary patient. Many treatments of the vascular or other systems entail the introduction of a device such as a stent, a catheter, a balloon, a wire guide, a cannula, a filter or the like.

For example, as described in U.S. Pat. No. 6,348,041, medical wire guides for vascular procedures, such as angioplasty procedures, diagnostic and interventional procedures, percutaneous access procedures, or radiological and neuroradiological procedures in general, traditionally comprise an elongated core element with one or more tapered sections near the distal end thereof and a flexible helical coil disposed about the distal portion of the core element. The distal extremity of the core element or a separate safety ribbon which is secured to the distal extremity of the core element extends through the flexible coil and is secured to the distal end member of the wire guide, which is a rounded member at the distal end of the helical coil. Torquing means are provided on the proximal end of the core element to rotate and steer the wire guide while it is being advanced through a patient's vascular system.

In order to follow the position of the wire guide within the body during a vascular procedure, it is desirable to use a material for the wire guide that is radiopaque for exposure to X-rays and MRI compatible for exposure to a magnetic field so as to make the tip portion of the wire guide visible on a screen. For example, the flexible helical coil of the wire guide may include a wire of radiopaque and/or MRI compatible material, or the distal end member of the wire guide may be designed as a coil of radiopaque and/or MRI compatible material that serves as a very soft and pliable tip member. The physician views the progress of the wire guide on a screen and makes the distal end of the wire guide enter and follow tortuous vascular vessels from the entry site through the various vascular branches to the target site by pushing and rotating the proximal end of the wire guide outside of the patient.

In connection with the advancement of the wire guide or once the wire guide has been positioned at the desired site, a wide variety of medical devices may be directed to the target site along the wire guide by simply sliding the device over the wire guide and advancing the device to the distal end of the wire guide. A typical medical device is a catheter, and very often a catheter and the wire guide are introduced in a common procedure where the wire guide is advanced a distance in front of the catheter, then the catheter is advanced over the wire guide, followed by a further advancement of the wire guide. Following placement of the catheter or other device, the wire guide can be removed if desired.

Once a catheter is placed in the vascular system, an embolization device can be positioned in a blood vessel to limit or stop the free flow of blood in an area of the blood vessel. As described in U.S. Pat. No. 6,776,788, embolization devices in the form of coils where a wire body is formed by a thread extending helically around the center line of the wire body are well-known. One example of an endovascular field of application for embolization devices for percutaneous, transluminal insertion using minimally invasive techniques, can be occlusion of an aneurism to prevent rupture of the vessel wall with resulting bleeding. Aneurisms may occur anywhere in the vascular system, but particularly cerebral and abdominal aneurisms require treatment to avoid life-threatening conditions. Another application is occlusion of arteriovenous malformations (AVM), where short-circuiting of arteries and veins looking like skeins of wool may occur, or occlusion of arteriovenous shunts or fistulas, which are major short-circuits between the artery side and the vein side in the vascular system with resulting heavy undesired blood flow. A fourth example of an application is blocking of the blood flow to a tumor, and a fifth, closure of traumatically conditioned blood flows owing to incised wounds or other bodily injuries or gastrointestinal bleeding.

In many current procedures, a wire guide mounted with the embolization device is pushed in through the catheter. When the wire body leaves the distal end of the catheter, it seeks to assume the predetermined shape. If the size and shape of the device are deemed suitable for the vessel geometry at the site of placement, the wire body is disconnected from the wire guide. Conventionally, the predetermined shape of the embolization device is made so that the wire body is helical with a helix diameter of the same size or slightly larger than the relevant vessel lumen, so that the wound wire body exerts an abutment pressure against the vessel wall. If the helix diameter is too large, the pressure against the vessel wall may become injuriously high, and if it is too small, the wire body can easily be released and drift away.

It is desirable to use a material for the embolization coil that is radiopaque to X-rays to indicate the position of the embolization coil once it is inserted in a blood vessel. In addition, since embolization coils remain in the vascular system for prolonged periods of time, it is desirable to use a material that is substantially free of nickel. Nickel ions are considered an undesired contaminant in the bloodstream of a patient because it is believed that they can lead to problems with cytotoxicity, carcinogenicity, genotoxicity and short to long term or chronic systemic toxicity. For example, it is known that nickel ions can elute from austenitic stainless steel implants creating problems in orthopedic patients contributing to tumor formation or artificial joint loosening and corrosion and wear.

It is known to use austenitic stainless steel alloy wire, such as double spring tempered 304V austenitic stainless steel, for the manufacture of implantable medical devices in order to obtain the desired strength, stiffness and flexibility for forming the wire into desired flexible shapes and navigating the arteriovenous malformations and other vascular lesions in small vessel anatomy. In addition, austenitic stainless steel alloys typically have a density close to 8 g/cm³, which renders them moderately radiopaque to allow a physician to view the progress of the device to a target site on a screen. However, austenitic stainless steel alloys are not very MRI compatible because they are ferromagnetic with a magnetic susceptibility in the neighborhood of 3000 x 10⁻⁶ SI. This means that a significant size artifact is created when the coil is subjected to a magnetic field during a MRI procedure. The artifact can potentially obscure the anatomical features of interest making a MRI guided diagnostic and or interventional embolization procedure very difficult to conduct. In addition, austenitic stainless steels contain a significant percentage of nickel, typically between 8 - 12% depending upon the grade. It is also known to use platinum based alloys, such as Pt - 8%W, Pt - Ni or Pt - Ir alloys, for the manufacture of implantable medical devices. These alloys have enhanced MRI compatibility and radiopacity compared to austenitic stainless steel alloys.

### Summary of the Invention

The present invention provides an improved implantable medical device comprising at least one portion made of a radiopaque material selected from the
group consisting of various palladium alloys having, by weight, rhenium in the range of 10 to 15 percent, and preferably rhenium at about 14 % of the alloy, by weight. The present invention further provides a wire guide, an embolization coil, a marker band, a stent, a filter, an RF ablation coil,
and an electrode having at least one portion made of the radiopaque material.

### Brief Description of the Drawing

Figure 1 shows an illustrative wire guide;
Figure 2 shows a partial, longitudinal section through the wire guide of Figure 1;
Figures 3-6 show partial, longitudinal views of embodiments of the wire guide of Figure 1 with a radiopaque marker made at least in part of a palladium based alloy according to the invention;
Figure 7 shows an illustrative embolization coil made at least in part of a palladium based alloy according to the invention;
Figure 8 shows a partially sectioned view of the embolization coil of Figure 7 in an elongated condition prior to being wound into a conically helically shaped coil; and
Figure 9 shows the longitudinally extending coil of Figure 8 wound around the tapered surface of a mandril to form a conically helically shaped coil.

### Detailed Description

The following provides a detailed description of currently preferred embodiments of the present invention. The description is not intended to limit the invention in any manner, but rather serves to enable those skilled in the art to make and use the invention.

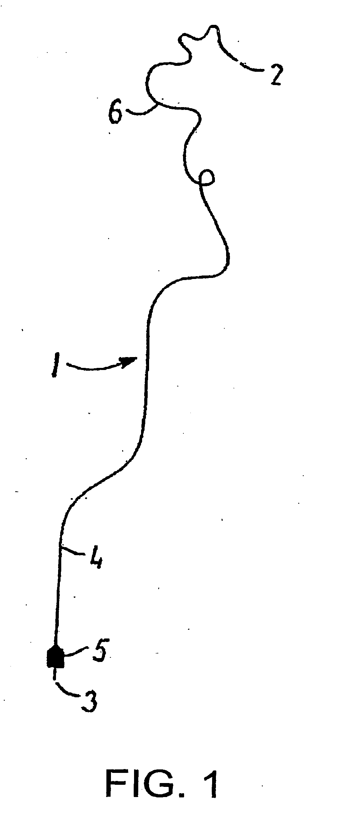

FIG.1 shows an illustrative medical wire guide denoted by 1 and having a distal end 2 capable of being advanced to a target site in the vascular system and a proximal end 3 that is kept outside the patient's body. A shaft portion 4 extends from the proximal end towards the distal end and carries near the proximal end a handle 5 releasably secured to the wire guide. The wire guide can typically have a length in the range of 30-600 cm and a maximum outer diameter in the range of 0.204-1.321 mm (0.008-0.052 inches). It can also include several segments where the proximal segment has a larger diameter than one or more intermediate segments which has/have larger diameters than the distal segment. When such a wire guide follows a tortuous vessel path involving several sharp bends, loops and the like, it is desirable that a turning of handle 5 results in a similar turning of the distal end 2.

The shaft portion 4 can include a solid shaft which is of a metallic material such as medical grade stainless steel or Nitinol. In that case a coiled distal portion 6 is fixed onto and in extension of the shaft portion. However, in the preferred embodiment the coiled portion continues from the distal to the proximal ends, and the use of a solid shaft is made superfluous. The coiled portion ends distally at the distal end 2, which is a member having a soft front end termination, such as a rounded front or a front of very flexible material or very flexible configuration. Distal end member 2 can be a solder, or a sphere that can be laser welded, for example, onto the distal end of the coiled distal portion 6.

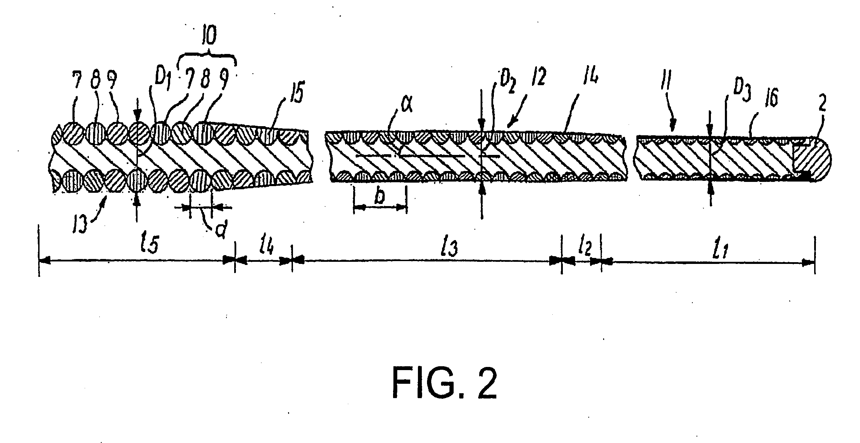

In the embodiment depicted in FIG. 2, a wound group of three wires 10 extends from a position at the shaft portion 4 to the distal end member 2. Three wires 7, 8 and 9 have been placed next to each other and have been wound in a common operation into the wound group of three wires 10 with a pitch angle α of the individual wire of about 40 degrees. The wire 7, 8 or 9 is of a linear elastic material, such as stainless steel, titanium or tantalum, or it is made of a superelastic alloy, such as Nitinol. The pitch angle is the included angle between the longitudinal axis of the wire guide and the center axis of the relevant wire 7, 8 or 9. The size of the pitch angle depends on the diameter of the wire, the diameter of the wire guide, and of the number of wires in a group. The most preferred pitch angle is in the range of 50 to 70 degrees. Also, if pushability is the most important criteria, the pitch angle can be chosen in the range of 35-50 degrees, for example. If bending flexibility is most important, the pitch angle can be chosen in the range of 70-76 degrees, for example. However, the combination of torque transferral, pushability and transverse flexibility is normally well-balanced for pitch angles in the range of 50-68 degrees. The wire guide is made by placing a group of from one to eight wires in a row next to each other, such as according to the desired pitch angle, after which the group of wires is wound about a mandrel. Then the mandrel with the coiled wires can be subjected to heat treatment in order to remove residual stresses from the wires. After the heat treatment the mandrel is removed from the coiled wires.

In order to make the tip portion of the wire guide 1 more visible on a screen it is desirable to use some kind of radiopaque material, such as a palladium based alloy which has equivalent or improved radiopacity, ultimate tensile strength and/or magnetic susceptibility compared to known platinum based alloys suitable for use in medical wire guides. For example, it is believed that a palladium alloy having, by weight, about 10 percent rhenium is preferable. This particular composition is biocompatible and hemocompatible and has the following material properties: a radiopacity approximately equivalent to known platinum alloys with 8 percent tungsten; a nominal ultimate tensile strength of about 1448 MPa (210 ksi), which ultimate tensile strength can be in the range of about 1379-1586 MPa (200-230 ksi), as compared to a nominal ultimate tensile strength of about 1379 MPa (200 ksi), which ultimate tensile strength can be in the range of about 1241-1586 MPa (180-230 ksi), for known platinum alloys with 8 percent tungsten; and a magnetic or bulk susceptibility of about 50x10⁻⁶ SI when measured using a Guoy balance, as compared to a magnetic or bulk susceptibility of about 250x10⁻⁶ SI when measured using a Guoy balance for known platinum alloys with 8 percent tungsten or a magnetic or bulk susceptibility of about 230x10⁻⁶ SI when measured using a Guoy balance for known platinum alloys with 10 percent iridium. By further comparison, known 304V austenitic stainless steel has an ultimate tensile strength of about 2069 MPa (300 ksi) and a magnetic or bulk susceptibility of about 3200x10⁻⁶ SI when measured using a Guoy balance, and known Inconel^{®} nickel chromium alloy 625 has an ultimate tensile strength of about 2069 MPa (300 ksi) and a magnetic or bulk susceptibility of about 1200x10⁻⁶ SI when measured using a Guoy balance.

It has been recognized by the present inventors that the high tensile strength properties of the palladium alloy may be improved by increasing the amount of rhenium in the composition or by modifying the drawing and annealing process for the wire. For example, it has been found that a palladium alloy having, by weight, about 12 percent rhenium approximately doubles the ultimate tensile strength (and the Brinell hardness) of palladium, whereas a palladium alloy having, by weight, about 15 percent rhenium approximately triples the ultimate tensile strength (and the Brinell hardness) of palladium. Further, it has been found that a palladium alloy having, by weight, about 14 percent rhenium also is preferable. This particular composition also is biocompatible and hemocompatible and has a radiopacity in the range of a platinum alloy with 8 percent tungsten, an ultimate tensile strength of about 1862 MPa (270 ksi), and a magnetic or bulk susceptibility of about 25x10⁻⁶ SI when measured using a Guoy balance.

Additionally, it has been found by the present inventors that a palladium alloy having, by weight, rhenium in the range of about 10 to 15 %, preferably 10 to 15 %, and more preferably in the range of about 11 to 14 %, preferably 11 to 14 %, produces an alloy with advantageous properties. Even more preferably, a palladium alloy having, by weight, rhenium at about 14 %, or 14 %, has been found to impart particularly advantageous properties over other alloys currently in use, as discussed above.

In addition, the following Table 1 shows palladium based alloys percentage shown by weight) suitable for use in wire guides, which are believed to offer equivalent or better material properties, including tensile strength, radiopacity and MR compatibility, compared to known platinum based alloys and austenitic stainless steel alloys. In particular, these palladium alloys are believed to exhibit higher tensile strength and at least equivalent radiopacity compared to known platinum alloys and austenitic stainless steel alloys.

As will be readily understood by the skilled person, the alloys of the present invention may consist exclusively of the specifically recited elements, such that the alloys are in a substantially pure form. In other words, palladium makes up the remainder of the recited alloy, to the exclusion of all other (non-recited) substituents. Alternatively, it is to be understood that the presently recited palladium alloys may also comprise other substituents as well as those specifically recited, as may advantageously be desired. Of course, in either situation the skilled person will also be aware that the recited alloys may also contain various impurities and other small amounts of matter, but in such amounts so as not to effect the advantageous properties of the inventive alloys. Preferably, such trace amounts of material will be present in less than e.g. 1000 ppm. One such element that may not be desired to be present in the present alloys is e.g. nickel. This has been found to be a potential allergen to those allergic to the metal, and thus is undesirable in medical implants which are to remain in the body for substantial amounts of time, e.g. embolization coils.

**Table 1**

| **Alloy** | **Density (g/cm³)** |
|---|---|
| CP Pd | 12 |
| Pd-xRe | 12-13.8 |
| (x = 0 to 20%) | |
| Pd-xRe-yW | 12 - 16 |
| (x = 0 to 20%, y = 0 to 30%) | |

Suitable ternary or quaternary palladium based alloys are possible by alloying palladium with two or three different elements. For example, as shown in Table 1, palladium may be alloyed with

rhenium in the range of up to about 20 percent and tungsten in the range of up to about 30 percent, to form a suitable alloy.

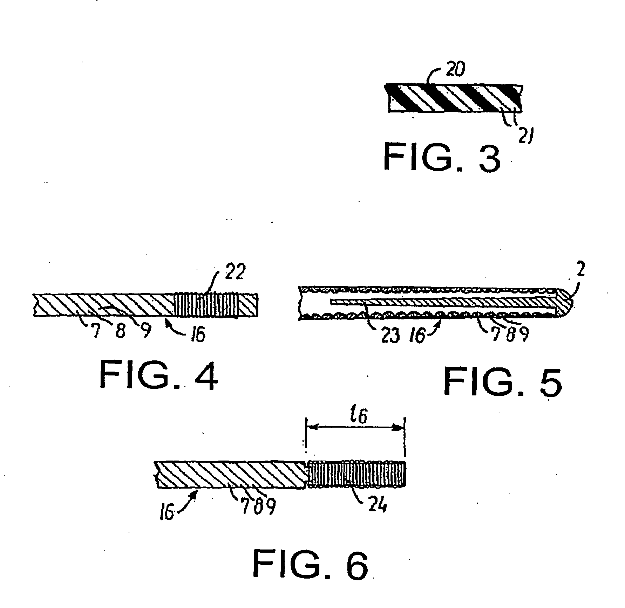

In the embodiment illustrated in FIG. 3 by a sectional view of the distal portion, one wire 20 or strand out of a group of three is of the palladium based alloys shown above in Table 1 and the remaining two 21 are of the high strength material. The wire 20 can have a relatively short length and be fixed in distal extension of a wire of the same type as wires 21. As an alternative, in the embodiment illustrated in FIG. 4, a wire guide distal portion 16 of the type shown in FIG. 2 can be provided with a coil 22 of wire made of the palladium based alloys shown above in Table 1. The coil 22 has a very small wire diameter, such as 0.05-0.35 mm. The coil has a pitch distance corresponding to the diameter of the wire, and consequently coil 22 is unable to transfer torque and is very flexible so that the desired properties of the distal wire guide portion are not impaired by adding coil 22 to the wire guide.

It will be understood by one of ordinary skill in the art that the wire guides described herein are intended as illustrative rather than limiting and that, according to the present invention, palladium based alloys are applicable to wire guides of different construction. For example, another embodiment according to the invention is outlined in FIG. 5 where distal end 2 is of the palladium based alloys shown above in Table 1 and includes a thread or a ribbon 23 of similar material that extends centrally into the hollow inner space in the wire guide to a free end. Alternatively, it is possible to position a distal end 2 designed as a very soft coil 24 of the palladium based alloys shown above in Table 1 in extension of wires 7, 8, 9 as outlined in FIG. 6: Such a coil can for example have a length 16 of about 35 mm. Apart from making the tip portion visible, coil 24 can also serve as a very soft and pliable tip member.

In another embodiment according to the invention, the use of palladium alloy material for the illustrative wire guide 1 can be extended by using a cored wire for the wire guide distal portion 16. In such a cored wire, the palladium alloy material could form a case on the outside of the wire while a secondary alloy, such as austenitic stainless steel, nickel titanium shape memory alloy(Nitinol) or a cobalt based superalloy such as L - 605, MP35N or Elgiloy/Conichrome, could serve as the core on the inside. The palladium alloy material would provide for improved radiopacity and MRI compatibility while the secondary alloy would provide for high yield strength, high ultimate tensile strength and high stiffness for desired bending flexibility and pushability. Alternatively depending upon the desired bending flexibility and pushability, one of these secondary alloys could form the case on the outside of the wire while the palladium alloy material forms the core on the inside. Further, it should be recognized that secondary alloys such as martensitic stainless steel, precipitation hardening stainless steel, duplex stainless steel, maraging steel, music wire, Cr - V wire or rocket/missile wire could also be used to form the core.

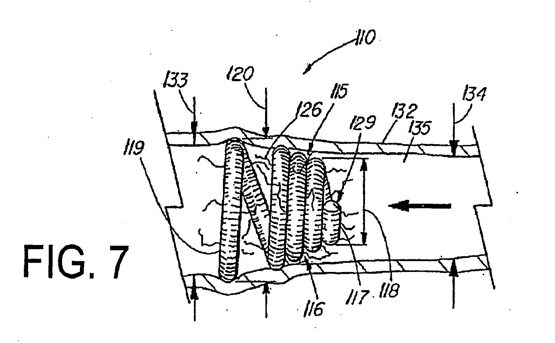

The wire guide 1 may be used to introduce an embolization coil through a catheter in a blood vessel to limit or stop the free flow of blood at an occlusion site. FIG. 7 depicts an illustrative embolization coil 110 longitudinally positioned in a vessel 132 of a human or animal body. The embolization coil 110 has been formed into a conically helically shaped coil 115 having a longitudinal axis that is substantially aligned with the longitudinal axis of the vessel 132. The conically helically shaped coil 115 has a plurality of radially expanding turns 116 with thrombogenic fibers 126 spaced at predetermined intervals along the length of the coil. The conically helically shaped coil 115 extends over almost the entire cross-sectional area of the vessel lumen 135 to substantially impede blood flow and attract thrombus. The thrombogenic fibers of the embolization coil 110 more readily attract thrombus to further build up a mass that entirely occludes the vessel 132.

Due to the build up of plaque and other irregularities in the vessel wall, the vessel lumen 135 has a maximum diameter 133 and a minimum diameter 134 through any given length of the vessel 132. To ensure longitudinal alignment and fixed positioning of the embolization coil 110 in the vessel lumen 135, a distal turn 117 of the conically helically shaped coil 11 5 has a minor diameter 118 that is less than the minimum diameter 134 of the vessel 132, and a compressed proximal turn 119 of the conically helically shaped coil 115, when in an uncompressed condition, has a major diameter 120 that is greater than maximum diameter 133 of the vessel 1 32. This sizing of the conically helically shaped coil 115 ensures that the distal end 129 of the coil is introduced from a delivery catheter into the central region of the vessel 132. The successive turns of the conically helically shaped coil 115 radially increase in size as the embolization coil 110 is released from the delivery catheter to engage and expand against the interior surface or intimal layer of the vessel wall. As a result, the minor diameter distal turn 117 does not engage the vessel wall and longitudinally displace the delivery catheter positioned at the occlusion site. The proximal radially expanding turns of the conically helically shaped coil 115 centrally and longitudinally position the coil 115 in the vessel lumen 135. Furthermore, the most proximal turns of the conically helically shaped coil 115 readily expand against the interior surface of the vessel wall and fixedly position the coil 115 at the desired occlusion site. Precision positioning of the conically helically shaped coil 115 is thus readily achieved without concern for the embolization coil 110 emerging from the delivery catheter and tumbling or drifting to an undesired occlusion site in the vessel.

Further, the embolization coil 110 may be mounted on the wire guide 1 using known coupling means for achieving greater control over the positioning of the embolization coil in the catheter. For example, a threaded connection between the embolization coil 110 and the wire guide 1 may include a thread provided on distal end 2 of the wire guide 1 for screwing into a socket or connector attached to the proximal end of the embolization coil 110.

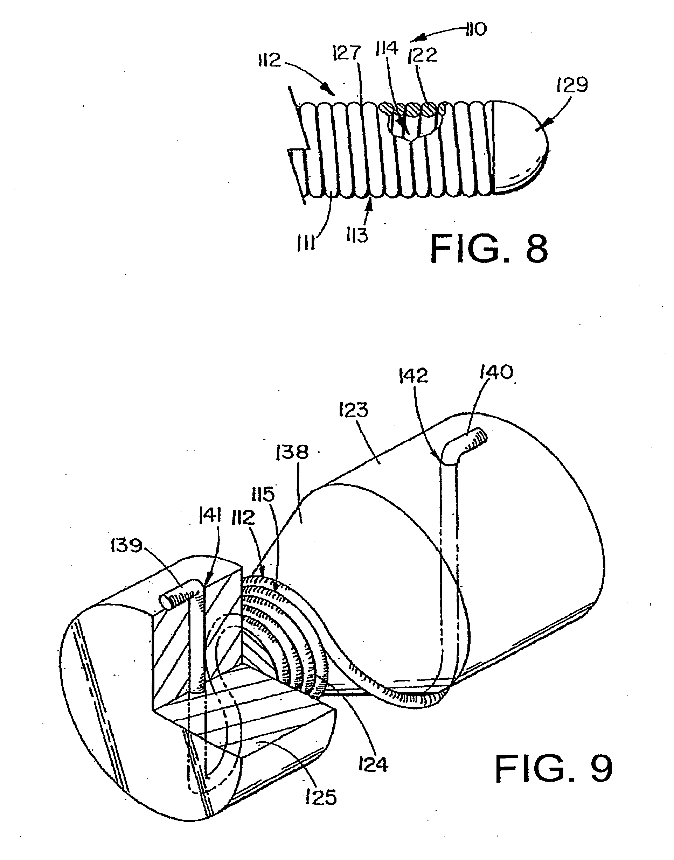

FIG. 8 depicts a partially sectioned view of the embolization coil 110 of FIG. 7 in an elongated condition prior to being wound into the conically helically shaped coil 115. The embolization coil 110 comprises a continuous wire strand 111 that has been wound in a well-known manner into a longitudinally extending coil 112 with a plurality of tightly spaced turns 113 and a hollow passage 114 extending longitudinally therein. For example, the continuous wire strand 111 is preferably wound into a longitudinally extending coil 112 approximately 6 cm long with a 0.356 mm (0.014") outside diameter and tightly spaced turns with minimal, if any, spacing 127 therebetween. Alternatively, the longitudinally extending coil 112 may be at least 6 cm long, perhaps about 6 to about 10 cm long, and the outside diameter can be at least 0.356 mm (0.014"). Distal end 129 of the longitudinally extending coil is soldered or welded to present a rounded or smooth surface, which will not catch on the interior surface of the guiding catheter. The longitudinally extending coil 112 may be wound around a longitudinally tapered surface 138 of a mandril 123 to form the conically helically shaped coil 115 with minimally spaced turns 124, as shown in FIG. 9.

Continuous wire strand 111 comprises a metallic alloy material 122. In order to make the embolization coil 110 more visible on a screen it is desirable to use some kind of radiopaque material, such as a palladium based alloy which has equivalent or improved ultimate tensile strength and radiopacity compared to known platinum based alloys suitable for use in embolizations coils. For example, it is believed that a palladium alloy material 122 having, by weight, about 10 percent rhenium is preferable. This particular composition has a radiopacity approximately equivalent to a platinum alloy with 8 percent tungsten, an ultimate tensile strength of about 1447.95 MPa (210 ksi), and a magnetic or bulk susceptibility of about 50x10⁻⁶ SI when measured using a Guoy balance, as described above. The composition is also biocompatible and hemocompatible. It should be recognized that the high tensile strength properties of this composition may be improved by increasing the amount of rhenium in the composition or by modifying the drawing and annealing process for the wire.

It is also believed that a palladium alloy material 122 having, by weight, about 14 percent rhenium is preferable. This particular composition also has a radiopacity in the range of a platinum alloy with 8 percent tungsten, an ultimate tensile strength of about 1861.65 MPa (270 ksi), and a magnetic or bulk susceptibility of about 25x10⁻⁶ SI when measured using a Guoy balance, as described above. This composition is also biocompatible and hemocompatible. It has been found that such an alloy composition does not substantially magnetise, and thus use of this alloy in MR applications is of particular benefit. Specifically, when this composition is drawn into the wire strand 111, the drawn wire has an ultimate tensile strength of about 1861.65 MPa (270 ksi) and an elongation to fracture of about 3 - 4%. Such a high ultimate tensile strength has not before been achieved using these and similar alloys. It has been surprisingly found by the present inventors that the hardening experienced by the inventive alloys may result from work hardening (i.e. cold reductions in excess of 90 - 95 %), preferably without any interpass recrystallization anneals. This creates a very elongated grain structure with pancake-shaped grains that exhibit a high aspect ratio. Examination of these grains at very high magnification with a TEM (transmission electron microscope) reveals a well-defined subgrain structure with a high dislocation density. It is this high dislocation density that may give the alloy wire in the cold drawn condition its high yield and ultimate tensile strength. These properties are desirable because testing has shown that if the ultimate tensile strength of the drawn wire exceeds approximately 1896 MPa (275 ksi), the drawn wire may fracture more easily when it is wound to form the embolization coil 110. Moreover, it is possible to subject the embolization coil 110 to a short term aging treatment to further improve its tensile strength properties after the drawn wire is wound and the shape of the embolization coil 110 is set. Such an aging process has been found to raise the ultimate tensile strength by 34.5-207 MPa (5 - 30 ksi). For example, testing has shown that an aging treatment performed at a temperature of about 699.82 Kelvin (800 degrees Fahrenheit) for a duration of about 20 minutes results in a wire having an ultimate tensile strength of about 2068 MPa (300 ksi) and an elongation to fracture of about a 2.5 - 3%.

It will be understood that it is possible to improve the tensile strength properties of the embolization coil 110 using other known methods, for example, precipitation hardening and solid solution strengthening. Further, it will be understood that it is possible to age the embolization coil 110 selectively, i.e., apply an aging treatment to portions of the embolization coil 110, in order to create a variable strength embolization coil. In this case, for example, the distal turn 117 of the embolization coil 110 would be softer and more pliable as compared to the proximal turn 119, or vice versa.

Table 2 is a summary of the magnetic susceptibility and ultimate tensile strength of alloys of the present invention compared to presently used alloys

**Table 2: the additional rhenium allows the alloy to be age or precipitation hardened as well as solid solution strengthened.**

| I.Alloy composition | Magnetic susceptibility II.(SI units) | III.Ultimate Tensile Strength IV. MPa (ksi) |
|---|---|---|
| 304 V SS | 3200 x 10⁻⁶ | 2069 (300) |
| Inconel alloy 625 | 1200 x 10⁻⁶ | 2069 (300) |
| Pt alloy (Pt - 8% W) | 250 x 10⁻⁶ | 1379 (200) |
| Pad - 10% Re | 50 x 10⁻⁶ | 1379-1586 (200-230) |
| Pd -14% Re | 2 5 x 10⁻⁶ | 1862-2069 (270-300) |

In addition, as noted above, the palladium based alloys shown above in Table 1 are believed to exhibit at least equivalent or improved ultimate tensile strength and radiopacity compared to platinum alloys. Furthermore, these palladium based alloys have densities that are approximately 40% less than traditional platinum alloys. Accordingly, embolization coils made from one of these palladium based alloys could have a smaller diameter than prior embolization coils made from platinum based alloys.

It will be understood by one of ordinary skill in the art that the embolization coils described herein are intended as illustrative rather than limiting and that, according to the present invention, palladium based alloys are applicable to embolization coils of different construction. For example, in another embodiment according to the invention, the use of palladium alloy material 122 for the illustrative embolization coil 110 can be extended by using a cored wire for continuous wire strand 111. In such a cored wire, the palladium alloy material 122 could form a case on the outside of the wire while a secondary alloy, such as austenitic stainless steel, nickel titanium shape memory alloy(Nitinol) or a cobalt based superalloy such as L - 605, MP35N or Elgiloy/Conichrome, could serve as the core on the inside. The palladium alloy material 122 would provide for improved radiopacity and MRI compatibility while the secondary alloy would provide for high yield strength, high ultimate tensile strength and high stiffness for recoil. Alternatively depending upon the desired spring properties, one of these secondary alloys could form the case on the outside of the wire strand 111 while the palladium alloy material 122 forms the core on the inside. Further, it should be recognized that secondary alloys such as martensitic stainless steel, precipitation hardening stainless steel, duplex stainless steel, maraging steel, music wire, Cr - V wire or rocket/missile wire could also be used to form the core.

Palladium alloys can advantageously be used in various applications of embolization coils to help treat e.g. aneurisms, or arteriovenous malformations in the brain. Since MRI is used routinely nowadays for scanning the brain, the present alloys present particular advantages over the alloys currently on the market (e.g. Pt-Ni or Pt-W alloys), since they have a smaller MRI 'footprint' and thus enable a clearer picture of the surrounding area to be obtained.

While the present invention has been described in terms of the above illustrative medical wire guides and embolization coils, it will be understood by one of ordinary skill in the art that, according to the present invention, palladium based alloys are applicable to other implantable medical devices, such as stents or filters, that are introduced temporarily or permanently in a body passage or lumen to treat a variety of medical conditions.

For example, in another embodiment according to the present invention, radiopaque marker bands for catheters are made from palladium based alloys as described herein. A marker band typically comprises a tubular body that is disposed at predetermined position on the exterior surface of a catheter. Such marker bands can be manufactured from seamless or welded and drawn tubing, which is made from one of the palladium based alloys listed in Table 1. Alternatively, radiopaque marker bands having a porous surface can be made from one of the palladium based alloys listed in Table 1 using a known metal foam manufacturing process, such as that used to manufacture trabecular metal by depositing chemical vapors onto a foam substrate. A porous maker band constructed using a metal foam manufacturing process would use less palladium by volume than marker bands made using seamless or welded and drawn tubing. Radiopaque marker bands also can be constructed from clad tubing that, according to the present invention, has a clad layer that is made from one of the palladium based alloys listed in Table 1. For example, powder particles of one of the palladium based alloys listed in Table 1 are deposited on the outer surface of a tubing of austenitic stainless steel, nickel titanium shape memory alloy (Nitinol) or a cobalt based superalloy such as L - 605, MP35N or Elgiloy/Conichrome, using known methods such that the palladium based alloy particles are bonded to the surface of the tubing. Radiopaque marker bands also can be constructed from one of the palladium based alloys listed in Table 1 using known ceramic or metal injection molding processes in which a powder of one of the palladium based alloys is mixed with a "binder" material such as plastic and then injected into a mold of the desired shape, for example a tubular body, which is then sintered at a higher temperature to consolidate the alloy powder. The "binder" material is then be removed from the molded part, for example, using standard solvent extraction techniques.

According to another embodiment, radiopaque RF ablation coils and radiopaque electrodes are made from palladium based alloys as described herein. RF ablation coils and electrodes used for treatment of atrial fibulation and atrial flutter and to map electrograms typically are made from a radiopaque and conductive material. The use of a radiopaque material allows a physician to guide the coils and electrodes to a target site. The use of a conductive material creates a conduction path for ablation of surrounding tissue.

For example, conventional radiofrequency catheter ablation techniques for treating a variety of cardiac arrhythmias typically use RF ablation coils made from platinum wire disposed about the tip of a catheter. The RF ablation coils are introduced within the body and are positioned at a desired site using the catheter. A physician can monitor the progress of the RF ablation coils in the body since the platinum renders them radiopaque. The ablation catheter can be maneuvered to various locations around the heart in a magnetic resonance imaging (MRI) system to acquire data for mapping electrograms. The ablation catheter is also used to position the RF ablation coil at the target site for treating the arrhythmia. Since the coil is conductive, a radiofrequency energy applied to the coil causes an electrical current to flow in the tissue surrounding the coil for ablating the target tissue.

As noted above, the palladium based alloys shown above in Table 1 are believed to offer equivalent or better material properties, including tensile strength, radiopacity and MR compatibility, compared to known platinum based alloys and austenitic stainless steel alloys. In addition, it is believed that these palladium based alloys exhibit similar heat transfer properties compared to known platinum alloys. For example, the thermal conductivity of palladium is approximately 0.168 cal/sq cm/cm/s degree C and the thermal conductivity of platinum is about 0.165 cal/sq cm/cm/s degree C. Furthermore, these palladium based alloys have densities that are approximately 40% less than traditional platinum alloys. Accordingly, RF ablation coils and electrodes made from one of these palladium based alloys could have a smaller diameter than prior RF ablation coils and electrodes made from platinum based alloys.

## Claims

1. An implantable medical device comprising: at least one portion made of a radiopaque material
wherein the radiopaque material is a palladium alloy having, by weight, rhenium in the range of 10 to 15%, preferably 11 to 14 %, preferably about14%.

2. The implantable medical device of claim 1, wherein the implantable medical device is selected from the group consisting of a wire guide, an embolization coil, a marker band, a stent, a filter, a RF ablation coil, and an electrode.

3. The implantable medical device of claim 2, wherein the implantable medical device is a wire guide which includes a coiled distal portion made of the radiopaque material.

4. The implantable medical device of claim 2, wherein the implantable medical device is an embolization coil including a coiled wire made at least in part of the radiopaque material, the coiled wire preferably comprising a conically helically shaped coil made of the radiopaque material, the coil having a plurality of radially expanding turns.

5. A process of manufacturing the implantable medical device of claim 4, comprising winding a continuous wire strand made from the alloy of claim 1 into a longitudinally extending, followed by winding said longitudinally extending coil around a longitudinally tapered surface to form a conically helically shaped coil.

6. The process of claim 5, further comprising the step of smoothing a distal end of the longitudinally extending coil.

7. A process for manufacturing an implantable medical device as recited in any of claims 1 to 4, said process comprising: selecting a palladium alloy; forming at least a portion of the implantable medical device from the palladium alloy; and after forming, hardening the portion of the implantable medical device to improve the tensile strength properties of the palladium alloy.

8. The process of claim 7 further comprising before forming, drawing the palladium alloy into a wire and, preferably winding the wire into the implantable medical device.

9. The process of claim 7 wherein the step of hardening is performed by work hardening without any interpass recrystallization anneals, and preferably comprises cold reductions in excess of 90 - 95%.

10. The process of claim 7 wherein the hardening is performed by an aging treatment and is preferably performed at a temperature of about 699.82 Kelvin (800 degrees Fahrenheit) for a duration of about 20 minutes.

11. The process of any of claims 7 to 10 wherein the hardening is performed selectively to the implantable medical device for providing a variable strength to the implantable medical device.

## Patentansprüche

1. Implantierbare medizinische Vorrichtung, die Folgendes umfasst: zumindest einen Abschnitt aus einem röntgenopaken Material, worin das röntgenopake Material eine Palladium-Legierung mit 10 bis 15 Gew.-%, vorzugsweise 11 bis 14 Gew.-% und vorzugsweise ca. 14 Gew.-% Rhenium ist.

2. Implantierbare medizinische Vorrichtung nach Anspruch 1, worin die implantierbare medizinische Vorrichtung aus der einen Führungsdraht, eine Embolisationsspule, ein Markierungsband, einen Stent, ein Filter, eine HF-Ablationsspule und eine Elektrode umfassenden Gruppe ausgewählt ist.

3. Implantierbare medizinische Vorrichtung nach Anspruch 2, worin die implantierbare medizinische Vorrichtung ein Führungsdraht ist, der einen gerollten distalen Abschnitt aus dem röntgenopaken Material aufweist.

4. Implantierbare medizinische Vorrichtung nach Anspruch 2, worin die implantierbare medizinische Vorrichtung eine Embolisationsspule mit einem aufgerollten Draht ist, der zumindest teilweise aus dem röntgenopaken Material besteht, wobei der aufgerollte Draht vorzugsweise eine Spule in Form einer konischen Spirale aus dem röntgenopaken Material umfasst, wobei die Spule eine Vielzahl von radial expandierenden Windungen aufweist.

5. Verfahren zur Herstellung der implantierbaren medizinischen Vorrichtung nach Anspruch 4, bei dem ein kontinuierlicher Drahtstrang aus der Legierung nach Anspruch 1 zu einer längs verlaufenden Spule gewickelt wird und anschließend die längs verlaufende Spule um eine in Längsrichtung verjüngte Oberfläche gewickelt wird, um eine Spule in Form einer konischen Spirale zu erhalten.

6. Verfahren nach Anspruch 5, das ferner den Schritt der Glättung eines distalen Endes der längs verlaufenden Spule umfasst.

7. Verfahren zur Herstellung einer implantierbaren medizinischen Vorrichtung nach einem der Ansprüche 1 bis 4, wobei das Verfahren folgende Schritte umfasst: Wahl einer Palladium-Legierung; Formung zumindest eines Abschnitts der implantierbaren medizinischen Vorrichtung aus der Palladium-Legierung; und nach der Formung Härtung des Abschnitts der implantierbaren medizinischen Vorrichtung zur Verbesserung der Zugfestigkeitseigenschaften der Palladium-Legierung.

8. Verfahren nach Anspruch 7, bei dem ferner die Palladium-Legierung vor dem Formen zu einem Draht gezogen wird und der Draht vorzugsweise zur implantierbaren medizinischen Vorrichtung gewickelt wird.

9. Verfahren nach Anspruch 7, worin der Härtungsschritt durch Kalthärtung ohne Zwischenrekristallisationstemperung durchgeführt wird und vorzugsweise Kaltreduktionen über 90-95% umfasst.

10. Verfahren nach Anspruch 7, worin die Härtung durch eine Alterungsbehandlung und vorzugsweise bei einer Temperatur von ca. 699,82 Kelvin (800 Grad F) für ca. 20 Minuten durchgeführt wird.

11. Verfahren nach einem der Ansprüche 7 bis 10, worin die Härtung selektiv auf der implantierbaren medizinischen Vorrichtung durchgeführt wird, um der implantierbaren medizinischen Vorrichtung variable Stärke zu verleihen.

## Revendications

1. Dispositif médical implantable comprenant au moins une partie réalisée en un matériau opaque au rayonnement, le matériau opaque au rayonnement étant un alliage de palladium qui présente en poids de 10 à 15 %, de préférence de 11 à 14 % et de préférence environ 14 % de rhénium.

2. Dispositif médical implantable selon la revendication 1, dans lequel le dispositif médical implantable est sélectionné dans l'ensemble constitué d'un guide-fil, d'une spirale d'embolisation, d'un ruban marqueur, d'une prothèse endovasculaire, d'un filtre, d'une spirale d'ablation à RF et d'une électrode.

3. Dispositif médical implantable selon la revendication 2, dans lequel le dispositif médical implantable est un guide-fil qui présente une partie distale en spirale réalisée en un matériau opaque au rayonnement.

4. Dispositif médical implantable selon la revendication 2, dans lequel le dispositif médical implantable est une spirale d'embolisation qui comprend un fil spiralé réalisé au moins en partie en le matériau opaque au rayonnement, le fil spiralé comprenant de préférence une spirale en forme d'hélice conique réalisée en le matériau opaque au rayonnement, la spirale présentant plusieurs tours qui s'agrandissent radialement.

5. Procédé de fabrication du dispositif médical implantable selon la revendication 4, comprenant l'enroulement d'un brin de fil continu réalisé en l'alliage selon la revendication 1 en une spirale qui s'étend longitudinalement, avec ensuite l'enroulement de ladite spirale s'étendant longitudinalement autour d'une surface effilée longitudinalement pour former une spirale configurée en hélice conique.

6. Procédé selon la revendication 5, comprenant en outre l'étape qui consiste à lisser l'extrémité distale de la spirale s'étendant longitudinalement.

7. Procédé de fabrication d'un dispositif médical implantable selon l'une quelconque des revendications 1 à 4, ledit procédé comprenant les étapes qui consistent à sélectionner un alliage de palladium, à former au moins une partie du dispositif médical implantable à partir de l'alliage de palladium et, après formation, à durcir la partie du dispositif médical implantable de manière à améliorer les propriétés de résistance en traction de l'alliage de palladium.

8. Procédé selon la revendication 7, comprenant en outre, avant le façonnage, l'étape qui consiste à étirer l'alliage de palladium en un fil et de préférence à enrouler le fil pour obtenir le dispositif médical implantable.

9. Procédé selon la revendication 7, dans lequel l'étape de durcissement est réalisée par écrouissage sans recuits de recristallisation intermédiaires et comprend de préférence des réductions à froid qui dépassent 90 à 95 %.

10. Procédé selon la revendication 7, dans lequel le durcissement est réalisé par traitement de vieillissement et est exécuté de préférence à une température d'environ 699,82 Kelvin (800 degrés Fahrenheit) pendant une durée d'environ 20 minutes.

11. Procédé selon l'une des revendications 7 à 10, dans lequel le durcissement du dispositif médical implantable est réalisé sélectivement pour conférer au dispositif médical implantable une résistance mécanique variable.
